# EUROPEAN PATENT SPECIFICATION

(11) **EP 1 691 873 B1**
(45) Date of publication and mention of the grant of the patent: **18.12.2013**
(21) Application number: 04819136.5
(22) Date of filing: 15.11.2004
(51) Int. Cl.: A61M 16/04

(54) **AIRWAY EXCHANGE CATHETER**
ATEMWEGSAUSTAUSCHKATHETER
CATHETER D'ECHANGE AERIEN

(30) Priority: 17.11.2003 US 520627 P; 07.10.2004 US 960457
(43) Date of publication of application: 23.08.2006
(73) Proprietor: Cook Medical Technologies LLC, Bloomington, IN 47404 (US)
(72) Inventor: GINGLES, Bruce, Bloomington, IN 47401 (US); SIROTA, Daniel, J., Bloomington, IN 47401 (US); HOFFA, Andrew, K., Bloomington, IN 47403 (US); FISCHER, Frank, J., Bloomington, IN 47401 (US); HUNT, James, B., Bloomington, IN 47401 (US)
(74) Representative: Exell, Jonathan Mark
(86) International application number: PCT/US2004/038181
(87) International publication number: WO 2005/049123

(56) References cited:
- WO-A-97/37702
- FR-A- 2 692 789
- US-A- 4 960 122
- US-A- 5 052 386
- US-A- 5 186 168
- US-A1- 2002 029 778
- US-B2- 6 623 449

## Description

### BACKGROUND

The present application relates generally to airway management devices, and more particularly, to an airway exchange catheter having a soft distal tip.

Airway exchange catheters are generally used for relatively uncomplicated and atraumatic endotracheal tube (ETT) exchange. The necessity to exchange an existing ETT in a patient for a new one may arise from, among other things, the physician's desire to utilize an ETT of a different size, the displacement of the existing ETT, or the malfunctioning of the existing ETT resulting from conditions such as blockage caused by patient mucous.

Proper placement and use of airway exchange catheters in ETT replacement is well known in the art, One particularly well-known method for replacing an ETT while maintaining ventilation of the patient is described in U.S. Patent No. 5,052, 386, incorporated by reference herein. According to this method, the existing ETT is disconnected from a ventilator, and an airway exchange catheter (referred to therein as an obturator tube) is connected to the ventilator by way of a removable connector. The ventilated airway exchange catheter is then inserted into the placed endotracheal tube. The airway exchange catheter is disconnected from the ventilator via the removable connector, and the ETT is removed from about the catheter. The replacement ETT is then inserted over the airway exchange catheter, and the catheter is reconnected to the ventilator utilizing the removable connector. Once the replacement ETT is properly positioned, the airway exchange catheter is removed and disconnected from the ventilator. The ventilator is then connected to the replacement ETT.
U.S. Patent No. 6,623,449 discloses a transformable catheter including a sheath having a length which is a substantial fraction of the entire length of the transformable catheter and a bore therethrough. A preformed inner catheter having a complex curve formed into the distal end thereof is disposed in the sheath bore. By axially moving the inner catheter with respect to the sheath, various tip shapes may be achieved. At least one wire runs from the proximal end of the sheath to the vicinity of the distal end of the sheath to selectively deflect the distal tip of the sheath as desired by the user to assist in reformation and transformation of the tip of the catheter. By suitable manipulation of the wire and of the inner catheter with respect to the sheath, the shape of the exposed portion of the distal end of the inner catheter may be transformed to any of a variety of shapes.
U.S. Patent No. 4,960,122 discloses a system for replacing a tracheal tube using an obturator that is inserted into the existing tube until it protrudes out from the distal end. The old tube is then slid out using the obturator as a guide. A new tube is inserted along the obturator until it has been properly inserted. The obturator is then removed. The obtuator has a flexible atraumatic tip at one end and a gripping section at the outer end. It is made from a flexible radiopaque material and has positioning marks to facilitate use with both endotracheal and tracheotomy tubes.

Although procedures for placement of an airway exchange catheter when replacing an ETT are well known, those skilled in the art will appreciate that the catheter is inserted into sensitive and crucial areas of the body's respiratory system. Improper positioning of an airway exchange catheter, or positioning of such a catheter in a patient whose respiratory system has been compromised in some manner, may cause serious consequences to the patient. One particular source of concern is the possibility of trauma or irritation to the tracheal carina or bronchial tree during insertion of the blunt distal tip of an airway exchange catheter. Airway exchange catheters generally are formed of a polymeric material having a relatively high durometer, and the hard distal ends can irritate the sensitive carina tissue. Since the catheters are inserted blindly, it is also possible to perforate the lung of a patient if the high durometer catheter is improperly inserted. In addition, improper positioning may result in excessive airway pressure, which may lead to pulmonary barotraumas in the patient.

It is desired to provide an airway exchange catheter for use in ETT replacement that overcomes the problems associated with prior art catheters, and particularly, a catheter that overcomes the problems caused by high durometer catheters.

### BRIEF SUMMARY

The above problems are addressed by the soft-tipped airway exchange catheter of the present invention.

According to an aspect of the present invention, there is provided a catheter for use in replacing an endotracheal tube as specified in claim 1.

In one form thereof, the less rigid distal portion comprises a material having a lower durometer than the material of the proximal portion. In another form thereof, the less rigid distal portion has a reduced thickness compared to the thickness of the proximal portion.

Other details of the invention and its preferred embodiments are provided in the following drawings, description and claims.

### BRIEF DESCRIPTION OF THE DRAWINGS

Fig. 1 illustrates an airway exchange catheter of the present invention shown placed in the ventilation passageway of an endotracheal tube that has been positioned in a patient;

Fig. 2 is a side view, partially in section, of an airway exchange catheter according to an embodiment of the present invention;

Figs. 3 and 4 illustrate alternative embodiments of a removable connector for connecting the airway exchange catheter to a ventilation apparatus;

Fig. 5 is a side view of another embodiment of an airway exchange catheter having a curved distal portion; and

Fig. 6 is a sectional view of another embodiment of an airway exchange catheter wherein the distal portion has a decreased wall thickness.

### DETAILED DESCRIPTION OF THE DRAWINGS AND THE PRESENTLY PREFERRED EMBODIMENTS

For the purposes of promoting an understanding of the principles of the invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

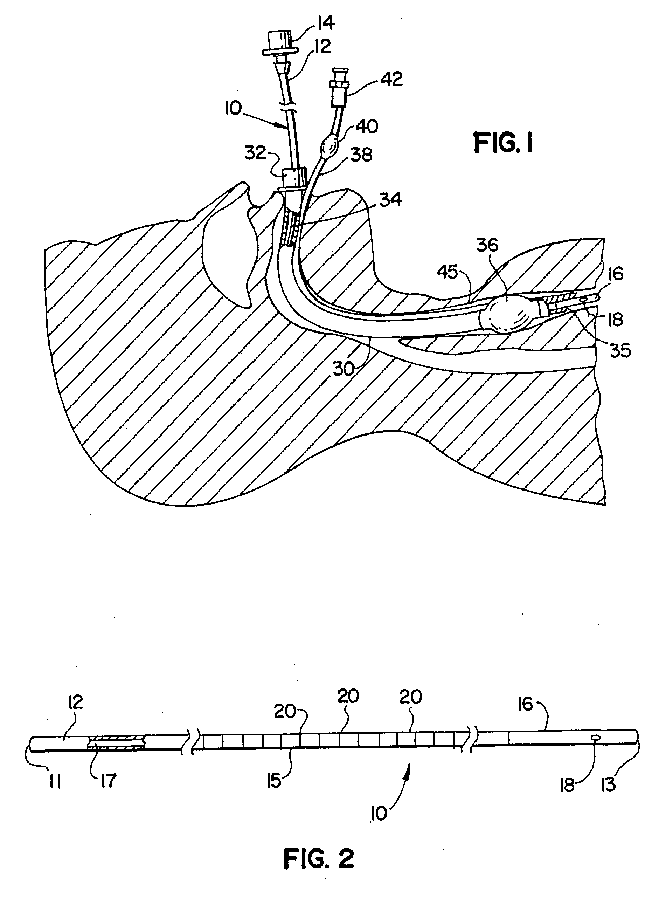

Fig. 1 depicts an illustrative airway exchange catheter 10 that is shown positioned in the ventilation passageway 34 of an endotracheal tube 30. Endotracheal tube 30 is shown positioned in conventional fashion in the trachea 45 of a patient through the mouth and airway of the patient. Airway exchange catheter 10 is used to provide ventilation to a patient during the replacement of an existing endotracheal tube with a new one.

Endotracheal tubes are well-known medical devices for providing ventilation to a patient. Endotracheal tube 30 includes a ventilator connector 32 at its proximal end for connecting tube 30 to a conventional ventilation apparatus (not shown), a ventilation passageway 34 extending therethrough for passage of a ventilating fluid, and an open distal end 35 through which the ventilating fluid passes to ventilate the patient. Open distal end 35 is shown partially broken away to better illustrate distal end 16 of airway exchange catheter 10. Endotracheal tube 30 typically includes an inflatable cuff 36 positioned in the vicinity of the distal end thereof to securely position and seal the distal end of the endotracheal tube in the trachea 45. Cuff 36 is inflated with air supplied through an inflation tube 38 attached to the external surface of endotracheal tube 30. An inflatable balloon 40 and an airtight connector 42 may be provided at the proximal end of the inflation tube for inflating the cuff and for providing a visual indication of the inflation of the cuff.

Fig. 2 is a side view, partially in section, of airway exchange catheter 10 according to the present invention. In the embodiment shown in Fig. 2, airway exchange catheter 10 includes proximal portion 12, intermediate portion 15 and distal portion 16. A fluid passageway 17 for passage of the ventilating fluid extends longitudinally through catheter 10 from an open proximal end 11 to an open distal end 13. Distal portion 16 includes one or more (preferably two) sideports 18. Sideports 18 are placed about 1 mm from the distal end of catheter 10 (only one of which is visible in the view of Fig. 2), and spaced 180 degrees from each other along the curvature of the catheter. Preferably, sideports 18 are elliptical or oval in shape to provide the largest port area without significantly weakening the structure of the catheter. The presence of sideports provides additional sites for ventilation of the patient in the event that distal end 13 of passageway 17 becomes blocked. Preferably, the proximal and distal ends of the airway exchange catheter are rounded as shown. The distal end is rounded to minimize trauma to the trachea of the patient, and the proximal end is rounded to improve the seal with a removable connector 14.

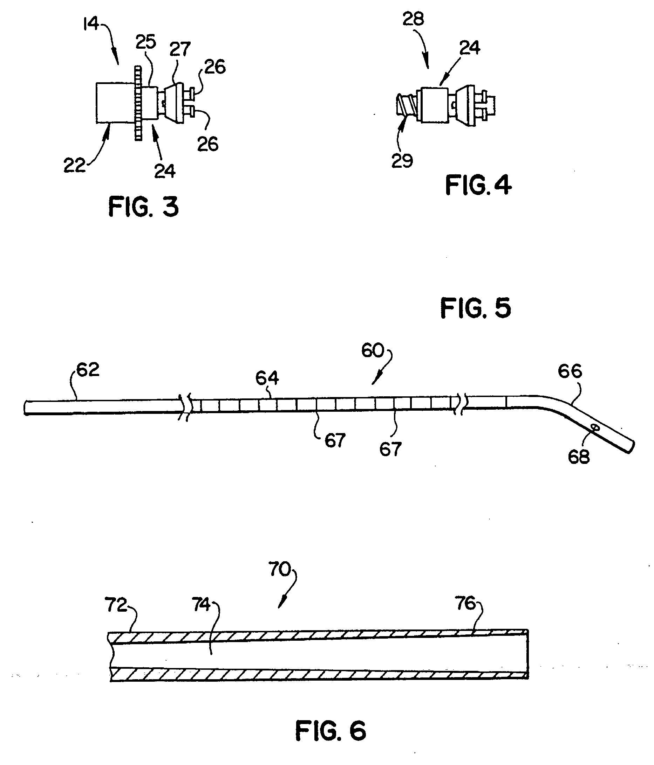

As shown in Fig. 1, a removable connector 14 is attached at the end of proximal portion 12 of the airway exchange catheter. Removable connector 14 is used to connect airway exchange catheter 10 to a conventional ventilation apparatus during replacement of the endotracheal tube. One example of a suitable removable connector 14 is shown in Fig. 3. This removable connector includes a conventional 15 mm ventilator fitting portion 22 for connection to a mating fitting of the ventilation apparatus, and a catheter fitting portion 24 for connection to the proximal end of the airway exchange catheter. Catheter fitting portion 24 can comprise virtually any biologically compatible material capable of forming a connection with an airway exchange catheter. In a preferred embodiment, catheter fitting portion 24 comprises a sleeve 25 having a plurality of fingers 26 axially-extending therefrom and aligned in a manner to comprise a chamber for snugly receiving catheter proximal end 12. A ring-like collar 27 is positioned about fingers 26 and axially movable between a proximal (open) position and a distal (closed) position to selectively disconnect and connect catheter 10 from removable connector 14.

Fig. 4 illustrates another example of a removable connector 28 for use with the inventive airway exchange catheter. Removable connector 28 is generally similar to removable connector 14, except that instead of the 15 mm ventilator fitting portion, connector 28 includes a Luer lock connector 29 for connection to a mating Luer lock connector on an auxiliary device, such as a ventilator.

Connectors 14 and 28 are preferably formed of a hard plastic material, such as polycarbonate. Particularly suitable connectors are the RAPI-FIT® connectors, available from Cook Incorporated, of Bloomington, Indiana.

Airway exchange catheters can be formed to have virtually any length. Generally, the length of the catheter will be significantly longer than the length of the endotracheal tube to be replaced, to permit easy handling of the catheter during the replacement procedure. Normally such catheters have a length between about 40 and 100 cm, the exact size being dependent in large part upon the size of the patient and the particular endotracheal tube used during the ventilation of the patient. Most conventional endotracheal tubes have an inner diameter between about 3 and 7 mm. Thus, in order to allow smooth passage of the airway exchange catheter through the endotracheal tube, an airway exchange catheter will typically have an outer diameter of no more than about 2.7 to 6.7 mm (8 to 20 French), or in other words, at least about 0.3 mm less than the inner diameter of the endotracheal tube. The inner diameter of the airway exchange catheter will typically be between about 1.5 and 6 mm. These dimensions are common in the industry, and are not intended to represent limitations to the present invention. Additional general details of airway exchange catheters, removable connectors, and the use of such catheters in replacing an endotracheal tube with a new tube are provided in the incorporated-by-reference U.S. Patent No. 5,052,386, and need not be repeated herein.

In the inventive airway exchange catheter, proximal portion 12 and intermediate portion 15 normally have either the same, or a similar, durometer. The term "durometer" is used here is its conventional sense to refer to the hardness of a material. A higher durometer indicates a harder material, whereas a lower durometer indicates a softer, more flexible material. Distal portion 16 has a lower durometer than the respective durometers of the proximal and intermediate portions. This results in a distal tip portion that is softer and more flexible than the proximal and intermediate portions. The soft tip results in less trauma to the tracheal carina or bronchial tree upon insertion of the device when compared to the use of a catheter having a higher durometer distal tip portion. It also reduces the possibility of perforating the lung during insertion of the airway exchange catheter.

For purposes of the present invention, the distal portion 16 will normally comprise approximately the distal 5-20 cm of airway exchange catheter 10. This distance is not critical, and a low durometer distal portion of greater or lesser dimension can be created as may be desired. The remaining length of the catheter comprises the proximal and intermediate portions. These portions may be broken down into any convenient proportion of the total length of the catheter, the intention being merely to comprise a main catheter body of higher durometer than the soft tip distal portion.

Although separate proximal and intermediate portions have been denoted on the drawings, this need not be the case. Rather, the catheter 10 may comprise only a proximal portion and a distal portion. In this instance, the high durometer proximal portion would preferably comprise the major length of the catheter, and the low durometer distal portion would preferably comprise a lesser length. However, if desired, the relative lengths can be reversed. In such a case, the high durometer proximal portion would comprise the lesser length, and the lower durometer distal portion would comprise the greater length.

In a particularly preferred embodiment, the proximal portion has a durometer between about 60 and 80, preferably between about 68 and 70, on the Shore D scale. The distal portion has a durometer between about 80 and 90, preferably about 85 on the Shore A scale. When an intermediate portion is present, the durometer of the intermediate portion is somewhere between that of the proximal and distal portions, preferably approximately midway between. However, the durometer of the intermediate section may be as high as that of the proximal section if desired.

Soft distal tip portion 16 may be bonded, or adhered, to the distal end of intermediate portion 14 by conventional means, such as thermal bonding or adhesion. Attachment of distal tips to elongated medical devices is well known in the art, and the skilled artisan can readily determine an acceptable attachment mechanism without undue experimentation. For example, the adjoining edges of the respective portions to be joined can be directly bonded to each other. Alternatively, the adjoining edges can be mutually tapered, slotted, or otherwise configured to mate in a manner to enhance the bonding therebetween. Although the airway exchange catheter may simply consist of two sections of different durometer bonded together, the invention is not so limited. Rather, the catheter can comprise more than two sections of different durometer bonded together. In such event, the sections are preferably aligned in order of decreasing durometer from the proximal end to the distal end, or in other words, from a harder proximal portion to a softer distal portion.

Airway exchange catheters are formed of well-known materials suitable for such use, preferably semi-rigid polymers such as polyester-based urethanes. In a preferred embodiment the proximal and intermediate sections are formed of a polyester-based urethane, and the distal section is formed from a softer material such as a polyether-based urethane. In another preferred embodiment, the polymeric catheter body is formed of radiopaque polyethylene. Conventional radiopaque agents can be added to other polymers to enhance the radiopacity of part, or all, of the catheter.

A particularly preferred way of forming the airway exchange catheter is by a continuous extrusion process. Continuous extrusion processes are known in the art and enable the continuous extrusion, without bonding, of a catheter having portions of different durometer. With continuous extrusion, a catheter can be extruded to be, e.g., rigid or semi-rigid at one end and soft at the other end. The catheter can be extruded to provide a gradual durometer decrease over a defined length of the catheter, or can be extruded to provide as many segments of different durometer as desired. With continuous extrusion, the catheter can be formed to eliminate areas of high stress that are common in many conventional devices at the bonding site where high and low durometer segments are joined together. Such high stress areas may result in breakage and possible separation of the two segments during usage of the device.

In addition to extruding sections of different durometer, continuous extrusion techniques can also be used to provide a device having segments that differ from one another in the type of polymer that is extruded, and in the level of radiopacity. Having segments of different levels of radiopacity in a device enables the physician to more clearly distinguish certain parts of the catheter from other parts under x-ray fluoroscopy. A preferred continuous extrusion process that is useful in preparing the airway exchange catheters of the present invention is a process referred to as Total Intermittent Extrusion (TIE), developed by Putnam Plastics Corporation of Dayville, Connecticut.

Preferably, airway exchange catheter 10 is also provided with a plurality of indicator markings 20 disposed at selected positions along the length of the catheter. In the embodiment of Fig. 2, markings 20 are provided at intermediate portion 15. However, if desired, markings may be distributed along the entire length of airway exchange catheter 10, or along one or more defined segments of the catheter. These indicators may be formed on the outer surface of the catheter with, for example, a non-radiopaque image black ink. The physician can visually observe these indicators during the insertion of the airway exchange catheter into the endotracheal tube to indicate relative positioning of the catheter in the patient. Further verification of the positioning of the catheter in the patient is provided with the radiopaque property of the catheter and visualization of the indicators under, for example, X-ray fluoroscopy.

Fig. 5 illustrates an alternative embodiment of an airway exchange catheter 60. The catheter of Fig. 5 has proximal 62 and intermediate 64 sections as in the embodiment of Fig. 2. However, the embodiment of Fig. 5 includes a curved distal tip 66 that has a lower durometer than the durometer of the remainder of catheter tube. Catheter 60 may also include indicator markings 67 and sideports 68. Airway exchange catheters having a curved distal tip are known in the art, and are used, for example, to assist in placement of an endotracheal tube in situations where there is inadequate exposure to the glottis. An example of a typical curved catheter is the FROVA™ intubating introducer, available from Cook Critical Care of Bloomington, Indiana. However, the airway exchange catheter 60 of the present invention differs from conventional curved catheters and introducers in that it is provided with a curved tip portion having a lower durometer (e.g. is softer) than the main body of the catheter.

Fig. 6 illustrates a sectional view of another alternative embodiment of the present invention. This embodiment illustrates a sectional view of a portion of a catheter 70 having proximal portion 72, distal portion 76, and a passageway 74 extending therethrough. In this embodiment, the wall of the catheter has a gradually decreasing thickness from proximal portion 72 to distal portion 76, thereby resulting in a less-thick distal portion 76 that has greater flexibility than proximal portion 72. The flexibility of this catheter distal tip provides benefits similar to those obtained by providing a low durometer tip as described above. The catheter wall need not have a gradually decreasing thickness throughout its entire length as shown in the drawing. Rather, if desired, the wall of the proximal portion can be of constant thickness, and the wall of the distal portion can also be of a constant, but reduced, thickness when compared to the proximal wall. Alternatively, the wall of the proximal portion can be of a constant thickness, and the wall of the distal portion can be of gradually reducing thickness toward the distal end. This can be accomplished by bonding a distal end portion of decreased, or decreasing, thickness onto a main shaft body of constant diameter. Alternatively, catheter 70 can be extruded to provide for varying wall thicknesses in a single extrusion, with either a transition section or a continuous taper.

In this embodiment, it is preferred that the segments of catheter 70 are formed of the same polymer, wherein the catheter composition differs only in thickness of the catheter wall from one portion of the catheter to another. However, this need not be the case, and the catheter 70 may comprise one or more polymers of the same, or different, durometers bonded or extruded together as described.

It is therefore intended that the foregoing detailed description be regarded as illustrative rather than limiting, and that it be understood that it is the following claims that are intended to define the scope of this invention.

## Claims

1. A catheter (10; 60; 70) for use in replacing an endotracheal tube (30) in a patient, comprising:
a polymeric catheter body sized for insertion into a passageway of said endotracheal tube for ventilating said patient during replacement of said endotracheal tube, said catheter body having a proximal portion (12; 62; 72), a distal portion (16; 66; 76) and a ventilation passageway (17; 74) extending longitudinally therein, said distal portion having a rigidity that is less than a rigidity of said proximal portion wherein said distal portion (16; 66; 76) comprises a material having a lower durometer than a material that comprises said proximal portion (12; 62; 72), and wherein said proximal portion (12; 62; 72) has a durometer between about 60 and 80D, and said distal portion (16; 66; 76) has a durometer of between about 80 and 90A.

2. The catheter of claim 1, wherein said catheter body (10; 60; 70) further comprises a portion (15; 64) intermediate said proximal (12; 62; 72) and distal portions (16; 66; 76), said distal portion comprising a material having a lower durometer than a material comprising said intermediate portion.

3. The catheter of any preceding claim, wherein said proximal portion (12; 62; 72) comprises a polyester-based urethane and said distal portion (16; 66; 76) comprises a polyether-based urethane.

4. The catheter of any preceding claim, wherein said distal portion (16; 66; 76) is bonded to said proximal portion (12; 62; 72).

5. The catheter of any preceding claim, wherein said polymeric catheter body is formed by continuous extrusion.

6. The catheter of any preceding claim, wherein said polymeric catheter body is formed by continuous extrusion of said portions of different durometer.

7. The catheter of any preceding claim, wherein said catheter includes at least one side port (18; 68) positioned at said distal end (13) for ventilating said patient during replacement of said endotracheal tube (30).

8. The catheter of any preceding claim, wherein said proximal portion (12; 62; 72) has a durometer between about 68 and 70D, and said distal portion (16; 66; 76) has a durometer of about 85A.

9. The catheter of any preceding claim, wherein a minor portion of said catheter body is disposed at an angle relative to a major portion of said body, and wherein said angled portion comprises the less rigid portion.

10. The catheter of any preceding claim, wherein the distal portion (16; 66; 76) of said polymeric catheter body has a reduced thickness compared to the thickness of said proximal portion (12; 62; 72) of said polymeric body.

11. The catheter of any preceding claim, wherein said catheter body has a gradually reduced thickness from said proximal portion (12; 62; 72) to said distal portion.

## Patentansprüche

1. Katheter (10; 60; 70) zur Verwendung beim Ersetzen eines Endotrachealtubus (30) in einem Patienten, umfassend:
einen polymeren Katheterkörper, der so bemessen ist, dass er in einen Durchgang des Endotrachealtubus eingeführt werden kann, um den Patienten während des Ersetzens des Endotrachealtubus zu beatmen, wobei der Katheterkörper einen proximalen Abschnitt (12; 62; 72), einen distalen Abschnitt (16; 66; 76) und einen sich in Längsrichtung darin erstreckenden Beatmungsdurchgang (17; 74) hat, wobei der distale Abschnitt eine Starrheit hat, die geringer als die Starrheit des proximalen Abschnitts ist, wobei der distale Abschnitt (16; 66; 76) ein Material umfasst, das einen niedrigeren Durometer-Wert als ein Material hat, das den proximalen Abschnitt (12; 62; 72) umfasst, und wobei der proximale Abschnitt (12; 62; 72) einen Durometer-Wert zwischen ungefähr 60 und 80D hat und der distale Abschnitt (16; 66; 76) einen Durometer-Wert von zwischen ungefähr 80 und 90A hat.

2. Katheter nach Anspruch 1, wobei der Katheterkörper (10; 60; 70) ferner einen Abschnitt (15; 64) zwischen dem proximalen (12; 62; 72) und dem distalen Abschnitt (16; 66; 76) umfasst, wobei der distale Abschnitt ein Material umfasst, das einen niedrigeren Durometer-Wert als ein Material hat, das den Zwischenabschnitt umfasst.

3. Katheter nach einem der vorhergehenden Ansprüche, wobei der proximale Abschnitt (12; 62; 72) ein Urethan auf Polyesterbasis umfasst und der distale Abschnitt (16; 66; 76) ein Urethan auf Polyetherbasis umfasst.

4. Katheter nach einem der vorhergehenden Ansprüche, wobei der distale Abschnitt (16; 66; 76) an den proximalen Abschnitt (12; 62; 72) gebondet ist.

5. Katheter nach einem der vorhergehenden Ansprüche, wobei der polymere Katheterkörper durch kontinuierliche Extrusion gebildet ist.

6. Katheter nach einem der vorhergehenden Ansprüche, wobei der polymere Katheterkörper durch kontinuierliche Extrusion der Abschnitte mit unterschiedlichem Durometer-Wert gebildet ist.

7. Katheter nach einem der vorhergehenden Ansprüche, wobei der Katheter mindestens einen Seitenanschluss (18; 68) aufweist, der an dem distalen Ende (13) positioniert ist, um den Patienten während des Ersetzens des Endotrachealtubus (30) zu beatmen.

8. Katheter nach einem der vorhergehenden Ansprüche, wobei der proximale Abschnitt (12; 62; 72) einen Durometer-Wert zwischen ungefähr 68 und 70D hat und der distale Abschnitt (16; 66; 76) einen Durometer-Wert von ungefähr 85A hat.

9. Katheter nach einem der vorhergehenden Ansprüche, wobei ein kleinerer Abschnitt des Katheterkörpers in einem Winkel bezüglich eines größeren Abschnitts des Körpers angeordnet ist und wobei der abgewinkelte Abschnitt den weniger starren Abschnitt umfasst.

10. Katheter nach einem der vorhergehenden Ansprüche, wobei der distale Abschnitt (16; 66; 76) des polymeren Katheterkörpers eine verringerte Dicke im Vergleich zu der Dicke des proximalen Abschnitts (12; 62; 72) des polymeren Körpers hat.

11. Katheter nach einem der vorhergehenden Ansprüche, wobei sich die Dicke des Katheterkörpers vom proximalen Abschnitt (12; 62; 72) zum distalen Abschnitt allmählich verringert.

## Revendications

1. Cathéter (10 ; 60 ; 70) destiné à être utilisé pour remplacer un tube endotrachéal (30) dans un patient, comprenant :
un corps de cathéter polymère dimensionné de manière à être inséré dans un passage dudit tube endotrachéal pour ventiler ledit patient au cours du remplacement dudit tube endotrachéal, ledit corps de cathéter ayant une portion proximale (12 ; 62 ; 72), une portion distale (16 ; 66 ; 76) et un passage de ventilation (17 ; 74) s'étendant longitudinalement à l'intérieur de celui-ci, ladite portion distale ayant une rigidité inférieure à une rigidité de ladite portion proximale, ladite portion distale (16 ; 66 ; 76) comprenant un matériau ayant une dureté mesurée au duromètre inférieure à celle d'un matériau comprenant ladite portion proximale (12 ; 62 ; 72), et ladite portion proximale (12 ; 62 ; 72) ayant une dureté mesurée au duromètre comprise entre environ 60 et 80D, et ladite portion distale (16 ; 66 ; 76) ayant une dureté mesurée au duromètre comprise entre environ 80 et 90A.

2. Cathéter selon la revendication 1, dans lequel ledit corps de cathéter (10 ; 60 ; 70) comprend en outre une portion (15 ; 64) entre lesdites portions proximale (12 ; 62 ; 72) et distale (16 ; 66 ; 76), ladite portion distale comprenant un matériau ayant une dureté mesurée au duromètre inférieure à celle d'un matériau comprenant ladite portion intermédiaire.

3. Cathéter selon l'une quelconque des revendications précédentes, dans lequel ladite portion proximale (12 ; 62 ; 72) comprend un uréthane à base de polyester et ladite portion distale (16 ; 66 ; 76) comprend un uréthane à base de polyéther.

4. Cathéter selon l'une quelconque des revendications précédentes, dans lequel ladite portion distale (16 ; 66 ; 76) est collée à ladite portion proximale (12 ; 62 ; 72).

5. Cathéter selon l'une quelconque des revendications précédentes, dans lequel ledit corps de cathéter en polymère est formé par extrusion continue.

6. Cathéter selon l'une quelconque des revendications précédentes, dans lequel ledit corps de cathéter en polymère est formé par extrusion continue desdites portions de différentes duretés mesurées au duromètre.

7. Cathéter selon l'une quelconque des revendications précédentes, dans lequel ledit cathéter comporte au moins un orifice latéral (18 ; 68) positionné au niveau de ladite extrémité distale (13) pour ventiler ledit patient au cours du remplacement dudit tube endotrachéal (30).

8. Cathéter selon l'une quelconque des revendications précédentes, dans lequel ladite portion proximale (12 ; 62 ; 72) présente une dureté mesurée au duromètre comprise entre environ 68 et 70D, et ladite portion distale (16 ; 66 ; 76) présente une dureté mesurée au duromètre d'environ 85A.

9. Cathéter selon l'une quelconque des revendications précédentes, dans lequel une portion mineure dudit corps de cathéter est disposée suivant un certain angle par rapport à une portion majeure dudit corps, et dans lequel ladite portion inclinée comprend la portion moins rigide.

10. Cathéter selon l'une quelconque des revendications précédentes, dans lequel la portion distale (16 ; 66 ; 76) dudit corps de cathéter en polymère présente une épaisseur réduite par comparaison avec l'épaisseur de ladite portion proximale (12 ; 62 ; 72) dudit corps en polymère.

11. Cathéter selon l'une quelconque des revendications précédentes, dans lequel ledit corps de cathéter présente une épaisseur se réduisant progressivement depuis ladite portion proximale (12 ; 62 ; 72) jusqu'à ladite portion distale.
